# EUROPEAN PATENT SPECIFICATION

(11) **EP 3 019 121 B1**
(45) Date of publication and mention of the grant of the patent: **17.05.2017**
(21) Application number: 14759303.2
(22) Date of filing: 08.07.2014
(51) Int. Cl.: A61F 2/14

(54) **INTRASTROMAL CORNEAL INSERT**
INTRASTROMALER HORNHAUTEINSATZ
INSERTION CORNÉENNE INTRASTROMALE

(30) Priority: 08.07.2013 IT BO20130352
(43) Date of publication of application: 18.05.2016
(73) Proprietor: Ring & Co. S.r.l., 40132 Bologna (IT)
(72) Inventor: MULARONI, Alessandro, I-76125 Trani (Bari) (IT); MARIOTTI, Marco, I-76125 Trani (Bari) (IT)
(74) Representative: Minghetti, Mauro
(86) International application number: PCT/IB2014/062944
(87) International publication number: WO 2015/004600

(56) References cited:
- WO-A1-2009/079726
- US-A- 4 842 599
- US-A- 5 876 439
- US-A- 5 944 752
- US-B1- 6 494 910

## Description

### TECHNICAL FIELD OF THE INVENTION

The present invention concerns an intrastromal corneal insert.

More in particular, the invention concerns an intrastromal corneal insert for correcting sight defects such as keratoconus, myopia, astigmatism, and others.

### STATE OF THE ART

In the field of refractive eye surgery, i.e. aimed at correcting refraction errors due to a defect in focusing the images on the retina, such as keratoconus, myopia, and others, it is known to use inserts, substantially shaped with the shape of an arc of circumference, that are inserted in the peripheral stroma between the corneal lamellae through radial incisions that are suitably performed for example with laser technology.

Such inserts are normally very thin and transparent, and are made in inert or biocompatible polymer material, for example polymethyl methacrylate (PMMA) or the like.

Once they have been inserted, the aforementioned inserts are positioned substantially concentric with respect to the cornea itself; if, for example, the implant is carried out for correcting keratoconus, the presence of the inserts in the corneal tissue determines tension towards the periphery of the cornea that contributes towards flattening the central area thereof, indeed where there is the keratoconus, so as to in any case maintain the positive asphericity of the cornea.

This operation normally makes the central corneal area more regular, allowing the patient to substantially see better.

Over time, such inserts can then be removed and replaced with other ones with different dimensions for example so as to obtain a stronger corrective effect.

In practice it has been found, however, that implanting the aforementioned inserts between the corneal lamellae can cause, in the tissue areas of the cornea immediately adjacent to the surfaces of each of the inserts themselves, phenomena of poor irroration or stagnation of organic fluids, and mainly a poor supply of oxygen, which, in the most critical situations, can lead to hypoxia or necrosis, even if only covering small areas.

These drawbacks, over time, inevitably have repercussions on the result of the operation and on the quality of vision of the patient.

US 5,876,439 discloses an intrastromal corneal insert comprising an element with the shape of an arc of circumference, with an angular extension less than 360°, and made in inert or biocompatible polymer material; the circumference-shaped element comprises a first through holes, suitable for allowing organic fluids to pass, having the respective axes substantially perpendicular to the plane defined by said element, and a second through holes, suitable for allowing organic fluid to pass, having the respective axes substantially parallel to the plane defined by said element; the first through holes are arranged alternately with the second through holes.

### PURPOSES OF THE INVENTION

The technical task of the present invention is therefore that of improving the state of the art.

In such a technical task, one purpose of the present invention is to devise an intrastromal corneal insert that makes it possible to avoid the drawbacks mentioned above.

Such a task and such purposes are all achieved with the intrastromal corneal insert according to the attached claim 1. Insofar as the terms "invention" and/or "embodiment" are used in the following, and/or features are presented as being optional, this should be interpreted in such a way that the only protection sought is that of the invention as claimed. The intrastromal corneal insert according to the invention, comprises an element with the shape of an
arc of circumference with a predetermined angular extension less than 360° and is made in inert or biocompatible polymer material, intended to be implanted in the stroma of the cornea for correcting sight defects, said element with the shape of an arc of circumference comprising first through holes, suitable for allowing organic fluids to pass, having the respective axes that are perpendicular or substantially perpendicular with respect to the plane defined by the element with the shape of an arc of circumference, and second through holes, suitable for allowing organic fluids to pass, having the respective axes parallel or substantially parallel to the plane defined by the element with the shape of an arc of circumference; the first through holes are arranged alternately with the second through holes and are equally spaced apart from one another from them, and/or the first through holes are in communication with the second through holes.

The dependent claims refer to advantageous embodiments of the invention.

### BRIEF DESCRIPTION OF THE DRAWINGS.

These and further advantages shall become clearer by any man skilled in the art from the following description and from the attached drawings, given as an example and not for limiting purposes, in which:
figure 1 is a plan view of an insert according to the present invention;
figure 2 is a cross-section view of the insert made according to the plane II-II of figure 1;
figure 3 is a cross-section view of the insert made according to the plane III-III of figure 1;
figure 4 is a cross-section view of the insert made according to the plane IV-IV of figure 1;
figure 5 is a cross-section of the insert made according to the plane V-V of figure 1;
figure 6 is a cross-section view of the insert made according to the plane VI-VI of figure 1;

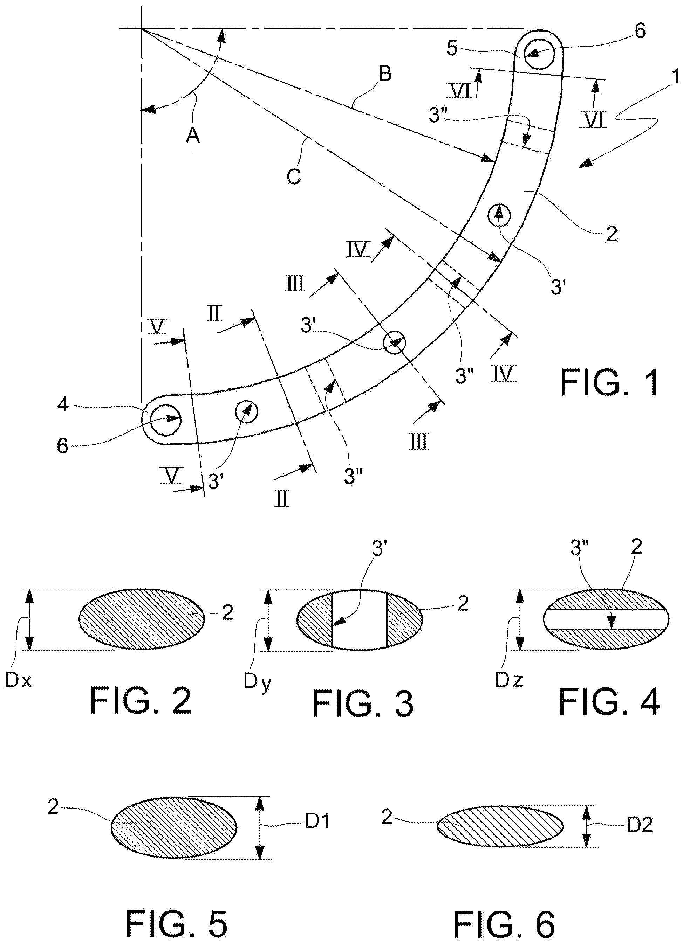

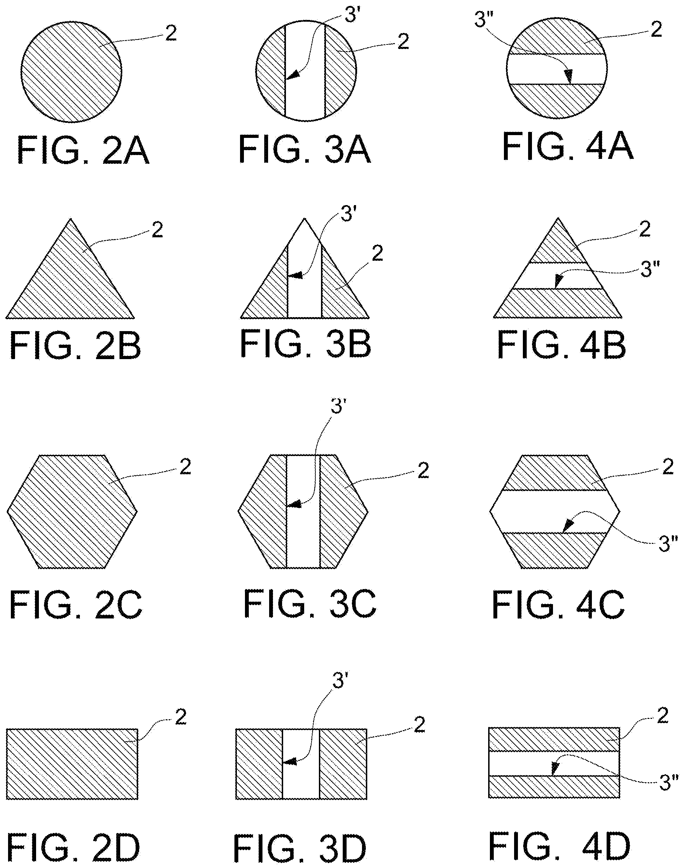

figures 2A,3A,4A are cross-section views of the insert according to another embodiment of the invention;
figures 2B,3B,4B are cross-section views of the insert according to yet another embodiment of the invention;
figures 2C,3C,4C are cross-section views of the insert according to yet another embodiment of the invention;
figures 2D,3D,4D are cross-section views of the insert according to a further embodiment of the invention;

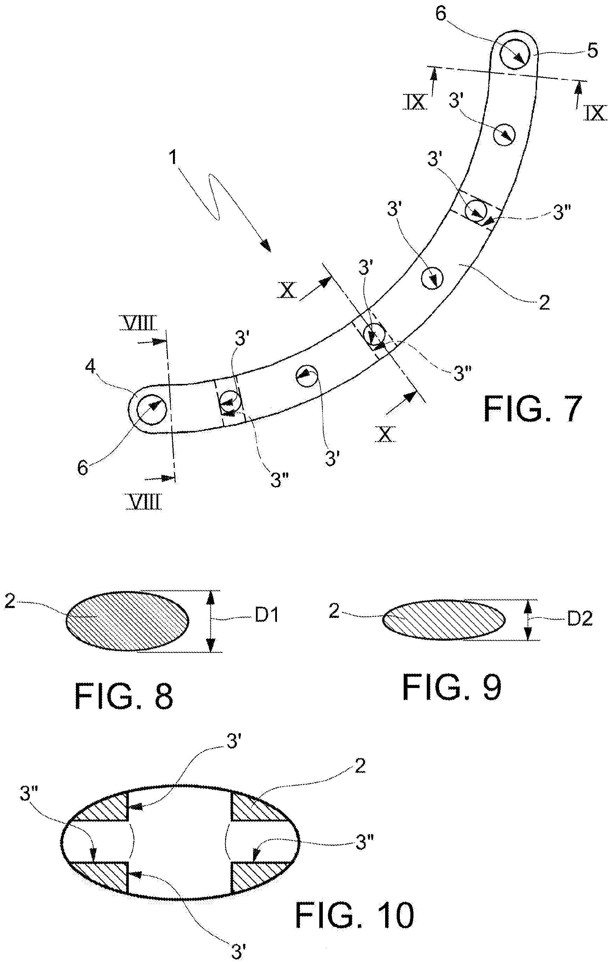

figure 7 is a plan view of an insert according to another embodiment of the invention;
figure 8 is a cross-section view of the insert made according to the plane VIII-VIII of figure 7;
figure 9 is a cross-section view of the insert made according to the plane IX-IX of figure 7;
figure 10 is a cross-section view of the insert made according to the plane X-X of figure 7;

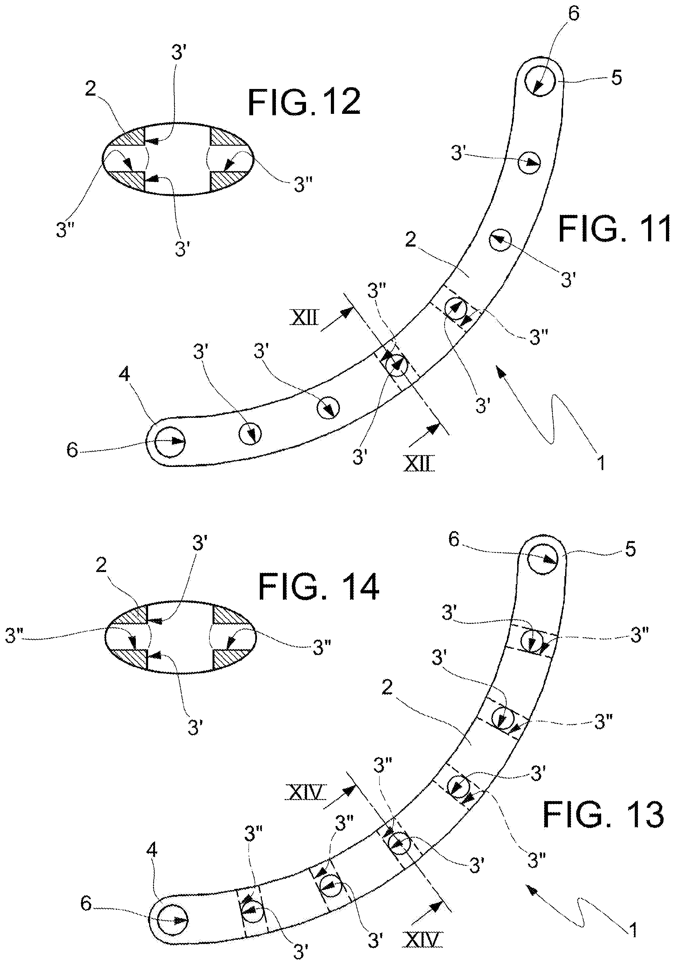

figure 11 is a plan view of an insert according to yet a further embodiment of the invention;
figure 12 is a cross-section view of the insert made according to the plane XII-XII of figure 11;
figure 13 is a plan view of an insert according to a further embodiment of the invention; figure 14 is a cross-section of the insert made according to the plane XIV-XIV of figure 13.

### EMBODIMENTS OF THE INVENTION.

With reference to figure 1, reference numeral 1 wholly indicates an intrastromal corneal insert according to the invention.

The insert 1 according to the present invention is particularly suitable for being used in refractive surgery treatments for correcting sight defects such as for example keratoconus, myopia, astigmatism, and others.

The indication of use in such treatments is in any case given completely as an example and not for limiting purposes, or rather the insert 1 according to the invention could be used also for other types of treatments.

The insert 1 comprises an element with the shape of an arc of circumference 2.

The element with the shape of an arc of circumference 2 has a predetermined angular extension A.

The angular extension A is less than 360°.

In the embodiment illustrated in figure 1 the angular extension A is, for example, of 90°.

Such an angular extension A could also have any other value, even above 90°, suitable for the specific application, without for this reason limiting the present invention.

The element with the shape of an arc of circumference 2 is made in inert or biocompatible polymer material.

For example, such an inert or biocompatible polymer material can comprise, or be made up of, polymethyl methacrylate (PMMA), or again from another polymer material with similar characteristics.

The element with the shape of an arc of circumference 2 can also be made in a mixture of different inert and biocompatible polymer materials.

The element with the shape of an arc of circumference 2 is transparent or substantially transparent, i.e. permeable to light.

The element with the shape of an arc of circumference 2 is intended to be implanted in the peripheral stroma, between the corneal lamellae, for correcting sight defects, as mentioned previously, according to known surgical methods and are not in any case object of the present invention, and that shall not therefore be described in detail.

According to one aspect of the present invention, the element with the shape of an arc of circumference 2 comprises at least one through hole 3',3".

The at least one through hole 3',3" is substantially foreseen in the central portion of the element with the shape of an arc of circumference 2.

The element with the shape of an arc of circumference 2 comprises a first end 4 and a second end 5 that are opposite one another.

By central portion we generally mean the area of the element 2 comprised between positioning end openings arranged at the opposite ends 4,5 of the element with the shape of an arc of circumference 2, as shall be described in greater detail in the rest of the description.

The at least one through hole 3',3" is advantageously suitable for allowing organic fluids, in the liquid or gas form, to freely pass from one side to the other of the element with the shape of an arc of circumference 2, for example from the upper side to the lower side and *vice versa,* but also from the inner side to the outer side and *vice versa,* as shall described in greater detail in the rest of the description.

In the embodiment of figure 1, the element with the shape of an arc of circumference 2 of the insert 1 comprises, more in detail, a plurality of through holes 3',3".

For example, in the embodiment of figure 1 the element with the shape of an arc of circumference 2 comprises six through holes 3',3".

There can however be any number of through holes 3',3" foreseen along the element with the shape of an arc of circumference 2, without particular limitations and in relation to the specific requirements of its application.

The through holes 3',3" are preferably angularly equally spaced apart from one another along the element with the shape of an arc of circumference 2, i.e. so as to be distributed homogeneously along the element 2 itself.

In some embodiments of the invention, however, the through holes 3',3" could be positioned at different distance from one another, so as to be, for example, more densely distributed in certain areas of the arc-shaped element 2, for specific requirements of greater passage of fluid.

In the embodiment of figure 1 the through holes 3',3" have a circular section.

In other embodiments, and in relation to specific requirements, the through holes 3',3" could also have a section that is different from the circular one.

In the embodiment represented in figures 1-6, and according to one aspect of the present invention, the element with the shape of an arc of circumference 2 comprises first through holes 3'.

The first through holes 3' have the respective axes perpendicular - or even substantially perpendicular - with respect to the plane defined by the element with the shape of an arc of circumference 2.

According to another aspect of the present invention, the element with the shape of an arc of circumference 2 comprises second through holes 3".

The second through holes 3" have the respective axes that are parallel - or even substantially parallel - to the plane defined by the element with the shape of an arc of circumference 2.

According to yet another aspect of the present invention, the first through holes 3' - having axes that are perpendicular to the plane of the element 2 - are arranged alternately with second through holes 3" - having its axis parallel to the plane of the element 2 - for a better distribution thereof and for a better circulation of organic fluids.

Thanks to this solution, the passage of organic fluids through the element with the shape of an arc of circumference 2 can occur, along the entire extension of the element with the shape of an arc of circumference 2, according to two directions that are substantially perpendicular to one another, making the circulation of organic fluid inside the corneal tissue extremely more effective.

In other embodiments of the invention, the first through holes 3' and the second through holes 3" can be arranged alternately in pairs, or rather there can be pairs of first holes 3' alternately with pairs of second holes 3".

Figures 2,3,4 illustrate the cross-section views of the insert 1 made respectively at the planes II-II, III-III and IV-IV indicated in figure 1.

Moreover, figures 5,6 illustrate the cross-sections of the insert 1 made respectively at the planes V-V and VI-VI of figure 1.

In the embodiment of figures 1-6, the cross-section of the insert 1 is elliptical.

However, the cross-section of the insert 1 can be of any shape, in relation to different application requirements.

For example, figures 2A,3A,4A refer to another embodiment of the insert 1 according to the invention, having a circular cross-section.

Figures 2B,3B,4B illustrate another embodiment of the insert 1 according to the invention, having a triangular cross-section, scalene or also isosceles.

Figures 2C,3C,4D illustrate another embodiment of the insert 1 according to the invention, having hexagonal cross-section.

Figures 2D,3D,4D refer to another embodiment of the insert 1 according to the invention having rectangular cross-section.

In other embodiments, the cross-section of the insert 1 can be semi-trapezoidal.

The first and second through holes 3',3" of the element with the shape of an arc of circumference 2 have a diameter preferably, but not exclusively, comprised between 0.1 mm and 0.3 mm.

The first holes 3' and the second holes 3" can have the same diameter, or diameters that are different from one another.

As a non-limiting example, in the embodiment of the invention according to figures 1-6 - but also, for example, in the embodiments of figures 2A,3A,4A,2B,3B,4B,2C,3C,4C,2D,3D,4D - the element with the shape of an arc of circumference 2 has an inner radius B of 2.80 mm, and an outer radius C of 3.20 mm.

In general, the dimensions of the inner radius B and of the outer radius C of the element with the shape of an arc of circumference 2 can be any, without limiting the present invention.

According to a further aspect of the present invention, the element with the shape of an arc of circumference 2 has a variable thickness passing from the first end 4 to the second end 5.

For example, the arc-shaped element 2 can have, at the first end 4, a first thickness D1, and at the second end 5, a second thickness D2, for example smaller than the first thickness D1, as shown in particular in figures 5 and 6.

The element with the shape of an arc of circumference 2 can pass from the first thickness D1 to the second thickness D2 in a linear fashion or substantially linear fashion.

In particular, figures 2,3,4 indicate the different thicknesses Dx,Dy,Dz of the respective cross-sections of the circumferential element 2, which can be for example increasing, if D1 is greater than D2, or decreasing, if D1 is smaller than D2.

As an alternative, in other embodiments, the element with the shape of an arc of circumference 2 can pass from the first thickness D1 to the second thickness D2 with a progression that is not linear, in relation to the specific application requirements.

This characteristic makes it possible to obtain different tensions in different areas of the peripheral stroma, which consequently induce different degrees of deformation of the stroma itself for corrective purposes.

In particular, the tension and the deformation of the stroma are greater in the areas at the first greater thickness D1.

Therefore, based upon the corrective effect that he desires to obtain the surgeon can position the insert 1 between the corneal lamellae so as to create the desired tensions in the required areas; for example, the surgeon will make sure that he positions the first end 4, having greater thickness D1, where it is necessary to have a stronger deformation of the cornea.

In conventional types of inserts, on the other hand, which have a constant thickness for their entire extension, there is a flattening of the cornea downstream of the implant that is constant for the entire length of the segment.

This criterion normally leads to improvements and regular changes in the cornea.

However, the corneal areas affected by pathologies often have thicknesses and curvatures that are different even in areas that are very close together.

The progression of the thickness D foreseen in the insert 1 according to the present invention, on the other hand, makes it possible to make each corneal area regular based upon the different curvatures and different thicknesses.

Moreover, it has been found that the presence of through holes 3',3" foreseen in areas of the insert 1 having different thickness makes it possible to ensure the necessary supply of organic fluids, with consequent optimal oxygenation, also in areas of the cornea in which pathologies have led to different thicknesses and curvatures, even if they are very close to one another.

In some embodiments of the invention, it could be foreseen for there to be a greater condensation of through holes 3,3" in the areas of the element with the shape of an arc of circumference 2 with smaller thickness, or in those with a greater thickness, so as to ensure a greater oxygenation of the tissue in such areas.

In brief, the presence of different thicknesses inside the same insert 1 makes it possible to carry out a personalised operation that considers the differences there can be in areas of the cornea that are even very close to one another.

Purely as an example, it should be noted that is some embodiments the first thickness D1 could be 0.5 mm, whereas the second thickness D2 could be 0.4 mm.

In other embodiments, the first thickness D1 could be 0.5 mm, whereas the second thickness D2 could be 0.3 mm.

In other embodiments, the first thickness D1 could be 0.4 mm, whereas the second thickness D2 could be 0.3 mm.

In yet other embodiments, the first thickness D1 could be 0.4 mm, whereas the second thickness D2 could be 0.2 mm.

In yet other embodiments, the first thickness D1 could be 0.3 mm, whereas the second thickness D2 could be 0.2 mm.

In other embodiments of the invention, which are not represented in the figures, the first thickness D1, which has a maximum value, can be substantially at the middle of the element with the shape of an arc of circumference 2.

The second thickness D2, that has minimum value, can be, on the other hand, at the first end 4 and at the second end 5 of the element with the shape of an arc of circumference 2.

The ends 4,5 of the element with the shape of an arc of circumference are preferably rounded, so as to facilitate implantation into the stroma of the cornea.

The element with the shape of an arc of circumference 2 of the insert 1 also comprises at least one end opening 6 that is suitable for a tool for manipulating the insert to be inserted 1.

As mentioned, such an end opening 6 is foreseen at one of the ends 4,5 of the element with the shape of an arc of circumference.

Preferably, the element with the shape of an arc of circumference 2 comprises two end openings 6 that are foreseen each at a respective end 4,5 of the element with the shape of an arc of circumference 2.

The presence of two end openings 6 allows the surgeon to grip the insert 1 from either end 4,5 of the element with the shape of an arc of circumference 2.

The end openings 6 are for example through openings, they have a circular section and have a diameter, for example, of 0.25 mm.

The end openings 6 could also have a section having a different shape, or have different dimensions, without any limitation.

Moreover, the end openings 6 could also not be through openings.

In some embodiments of the invention, there may be no end openings 6.

In the case in which such end openings 6 are present, by central portion of the element with the shape of an arc of circumference 2 we mean the portion comprised between the aforementioned end openings 6.

The invention thus conceived makes it possible to obtain important technical advantages.

Indeed, the first and the second through holes 3',3" make it possible for organic fluids - in the liquid or gas form - to freely pass substantially along the entire length of the element with the shape of an arc of circumference 2, both from the upper side to the lower side and *vice versa,* and from the inner side to the outer side, and *vice versa.*

In particular, this means that the aforementioned organic fluids can freely pass, for example, from one corneal lamella to another, thus preventing areas of the latter, even of considerable size, from suffering from problems of hypoxia, or other similar problems related to poor circulation of the fluids themselves.

In other words, the fluid can pass also according to directions that are substantially parallel with respect to the ideal surfaces on which the corneal lamellae are located.

Moreover, the presence of the through holes 3',3" according to two directions that are perpendicular to one another makes the insert 1 more flexible and manageable, which is advantageous when being implanted in the cornea of the patient.

The first and the second through holes 3',3" - equidistant and distributed along the element with the shape of an arc of circumference 2 alternately according to directions that are perpendicular to one another - have a completely innovative function, since they constitute the metabolic way of exchanging gases, fluids and nutrition between the corneal lamellae separated by the insert 1.

This promotes the normal corneal metabolism and prevents areas of corneal hypoxia from forming which could lead to the death of the cell tissue (corneal melting), which is the most frequent cause of surgery failure.

When such an even happens, the insert 1 must be removed.

It is thus ensured for there to be a normal trophism of the corneal tissue, avoiding hypoxia.

Together with the differentiated thickness of the element with the shape of an arc of circumference 2, which makes the corneal curvature micrometrically regular, a longer corneal life is ensured, preventing the probable requirement of corneal transplantation if the cornea itself loses endothelial cells following the lack of oxygenation.

Another embodiment of the insert 1 according to the invention is illustrated in figures 7-10.

This embodiment is different from the previous one, according to figures 1-6 for the fact that the element with the shape of an arc of circumference 2 comprises further first through holes 3' that are in communication with the second through holes 3", as illustrated in particular in figure 10.

In other words the further first through holes 3' intercept the second through holes 3".

This makes it possible to obtain, in some specific areas of the arc-shaped element 2 for example areas that are angularly equally spaced apart from one another - the free passage of the organic fluids themselves both from the upper side to the lower side of the element 2 and *vice versa,* and from the inner side to the outer side of the element 2 itself and *vice versa*.

Such a solution makes it possible to considerably increase the permeability of the insert 1 to organic fluids in some specific areas of the element with the shape of an arc of circumference 2, and in many directions.

The cross-section of the element with the shape of an arc of circumference 2 can have any of the shapes mentioned for the previous embodiments, or even other shapes.

The thickness of the element with the shape of an arc of circumference 2 can vary from the first end 4 to the second end 5 as described for the previous embodiment. another embodiment of the insert 1 according to the invention is illustrated in figures 11,12.

This embodiment is different from the previous one for the fact that the second through holes 3" are foreseen only at the middle of the element with the shape of an arc of circumference 2, whereas in the two most peripheral areas that are adjacent to the aforementioned middle line only first through holes 3' are foreseen.

The second through holes 3" are for example in a number of two.

Moreover, it is foreseen for there to be further first through holes 3' that intercept the second through holes 3", or rather that communicate with them.

This solution makes it possible to obtain the free passage of the same organic fluids both from the upper side to the lower side of the element 2 and *vice versa*, and from the inner side to the outer side of the element 2 itself, only at one preferential area of the element 2, or rather the middle area, so as to better control the phenomenon.

Also in this embodiment the element with the shape of an arc of circumference 2 has a variable thickness, according to the characteristics already mentioned concerning the embodiment of figures 1-6.

Moreover, also the cross-section of the element with the shape of an arc of circumference 2 can be any shape, for example elliptical or again according to what has been illustrated in figures 2A-2D, 3A-3D, 4A-4D.

Another embodiment of the invention is illustrated in figures 13-14.

In this embodiment, the element with the shape of an arc of circumference 2 comprises first through holes 3' and second through holes 3" that all communicate with one another.

In other words, each first through hole 3' is intercepted by a respective second through hole 3", as illustrated in particular in figure 14.

According to this embodiment, therefore, along the entire extension of the element with the shape of an arc of circumference 2 it is possible to obtain the free passage of the organic fluid themselves both from the upper side to the lower side of the element 2 and *vice versa,* and from the inner side to the outer side of the element 2 itself, and *vice versa*.

The cross-section of the element with the shape of an arc of circumference 2 can have any of the shapes mentioned for the previous embodiments, or even other shapes.

The thickness of the element with the shape of an arc of circumference is variable from the first end 4 to the second end 5 as described for the previous embodiments.

This solution of course makes it possible to maximise and optimise the circulation of the organic fluid through the insert 1, so as to achieve all the advantages mentioned previously in an even more effective manner.

Moreover, the flexibility and the manageability of the insert 1 are maximised.

It has thus been seen how the invention achieves the proposed purposes.

The present invention has been described according to preferred embodiments, but equivalent variants may be conceived without for this reason departing from the scope of protection offered by the following claims.

## Claims

1. Intrastromal corneal insert (1), comprising an element with the shape of an arc of circumference (2) with a predetermined angular extension (A) less than 360° and made in inert or biocompatible polymer material, intended to be implanted in the stroma of the cornea for correcting sight defects, said element with the shape of an arc of circumference (2) comprising first through holes (3'), suitable for allowing organic fluids to pass, having the respective axes perpendicular or substantially perpendicular to the plane defined by said element with the shape of an arc of circumference (2), and second through holes (3"), suitable for allowing organic fluids to pass, having the respective axes parallel or substantially parallel to the plane defined by said element with the shape of an arc of circumference (2), **characterised in that** said first through holes (3') are arranged alternately with second through holes (3"), and/or said first through holes (3') are in communication with said second through holes (3").

2. Insert according to claim 1, wherein said first through holes (3') and said second through holes (3") communicating with one another are foreseen at the middle of said element with the shape of an arc of circumference (2).

3. Insert according to claim 1, wherein said first through holes (3') and said second through holes (3") communicating with one another are foreseen along the entire extension of said element with the shape of an arc of circumference (2).

4. Insert according to one of the previous claims, wherein said element with the shape of an arc of circumference (2) has a variable thickness (D1, D2, Dx, Dy, Dz) from its first end (4) to its second end (5).

5. Insert according to claim 4, wherein said thickness (D1, D2, Dx, Dy, Dz) progressively varies in a linear, or substantially linear fashion, from said first end (4) to said second end (5).

6. Insert according to the previous claim, wherein said element with the shape of an arc of circumference (2) has a first thickness (D1) at said first end (4), and a second thickness (D2), less than (D1), at said second end (5).

7. Insert according to claim 4, wherein said element with the shape of an arc of circumference (2) has a first thickness (D1) substantially at its middle, and a second thickness (D2), less than (D1), at said first end (4) and second end (5).

8. Insert according to any one of the previous claims, wherein said first through holes (3') and said second through holes (3") are equally spaced apart from one another.

9. Insert according to one of claims 4-7, wherein said through holes (3',3") are more densely distributed in the area of said element with the shape of an arc of circumference (2) having greater thickness (D1,D2,Dx,Dy,Dz), or in the area of said element with the shape of an arc of circumference (2) having smaller thickness (D1,D2,Dx,Dy,Dz).

10. Insert according to any one of the previous claims, wherein said through holes (3';3") have a diameter of between 0.1 mm and 0.3 mm.

11. Insert according to any one of the previous claims, wherein said element with the shape of an arc of circumference (2) comprises at least one end opening (6), foreseen at said first end (4) and/or second end (5), suitable for a tool for manipulating the insert to be inserted.

12. Insert according to any one of the previous claims, wherein said element with the shape of an arc of circumference (2) has a cross-section in a shape selected from elliptical, circular, triangular, quadrangular, hexagonal, semi-trapezoidal, and the like.

13. Insert according to any one of the previous claims, wherein said inert or biocompatible polymer material comprises polymethyl methacrylate.

14. Insert according to any one of the previous claims, wherein said predetermined angular extension is of 90°.

## Patentansprüche

1. Intrastromales Hornhautimplantat (1), umfassend ein Element mit der Form eines Umfangsbogens (2) mit einer vorgegebenen Winkelerstreckung (A) von weniger als 360° und hergestellt aus einem inerten oder biokompatiblen Polymerwerkstoff, das dazu bestimmt ist, in das Stroma der Hornhaut implantiert zu werden, um Sehfehler zu korrigieren, wobei das Element mit der Form eines Umfangsbogens (2) erste Durchgangslöcher (3') umfasst, die dazu geeignet sind, organische Flüssigkeiten passieren zu lassen und deren jeweilige Achsen senkrecht oder im Wesentlichen senkrecht zu der Ebene verlaufen, die durch das Element mit der Form eines Umfangsbogens (2) definiert ist, und zweite Durchgangslöcher (3"), die dazu geeignet sind, organische Flüssigkeiten passieren zu lassen und deren jeweilige Achsen parallel oder im Wesentlichen parallel zu der Ebene verlaufen, die durch das Element mit der Form eines Umfangsbogens (2) definiert ist, **dadurch gekennzeichnet, dass** die ersten Durchgangslöcher (3') abwechslungsweise zu zweiten Durchgangslöchern (3") angeordnet sind und/oder die ersten Durchgangslöcher (3') mit den zweiten Durchgangslöchern (3") in Verbindung stehen.

2. Implantat nach Anspruch 1, wobei die ersten Durchgangslöcher (3') und die zweiten Durchgangslöcher (3"), die miteinander in Verbindung stehen, in der Mitte des Elements mit der Form eines Umfangsbogens (2) vorgesehen sind.

3. Implantat nach Anspruch 1, wobei die ersten Durchgangslöcher (3') und die zweiten Durchgangslöcher (3"), die miteinander in Verbindung stehen, längs der gesamten Erstreckung des Elements mit der Form eines Umfangsbogens (2) vorgesehen sind.

4. Implantat nach einem der vorstehenden Ansprüche, wobei das Element mit der Form eines Umfangsbogens (2) von seinem ersten Ende (4) zu seinem zweiten Ende (5) eine variable Dicke (D1, D2, Dx, Dy, Dz) aufweist.

5. Implantat nach Anspruch 4, wobei die Dicke (D1, D2, Dx, Dy, Dz) von seinem ersten Ende (4) zu seinem zweiten Ende (5) in linearer oder im Wesentlichen linearer Weise zunehmend variiert.

6. Implantat nach dem vorstehenden Anspruch, wobei das Element mit der Form eines Umfangsbogens (2) an dem ersten Ende (4) eine erste Dicke (D1) und an dem zweiten Ende (5) eine zweite Dicke (D2) aufweist, die geringer als (D1) ist.

7. Implantat nach Anspruch 4, wobei das Element mit der Form eines Umfangsbogens (2) im Wesentlichen in seiner Mitte eine erste Dicke (D1) und an seinem ersten Ende (4) und an seinem zweiten Ende (5) eine zweite Dicke (D2) aufweist, die geringer als (D1) ist.

8. Implantat nach einem der vorstehenden Ansprüche, wobei die ersten Durchgangslöcher (3') und die zweiten Durchgangslöcher (3") gleichmäßig voneinander beabstandet sind.

9. Implantat nach einem der Ansprüche 4 bis 7, wobei die Durchgangslöcher (3',3") dichter verteilt sind im Bereich des Elements mit der Form eines Umfangsbogens (2) mit der größeren Dicke (D1, D2, Dx, Dy, Dz) oder im Bereich des Elements mit der Form eines Umfangsbogens (2) mit der kleineren Dicke (D1, D2, Dx, Dy, Dz).

10. Implantat nach einem der vorstehenden Ansprüche, wobei die Durchgangslöcher (3';3") einen Durchmesser zwischen 0,1 mm und 0,3 mm aufweisen.

11. Implantat nach einem der vorstehenden Ansprüche, wobei das Element mit der Form eines Umfangsbogens (2) wenigstens eine Stirnöffnung (6) aufweist, die an dem ersten Ende (4) und/oder an dem zweiten Ende (5) vorgesehen und zum Einbringen eines Werkzeugs zum Handhaben des Implantats geeignet ist.

12. Implantat nach einem der vorstehenden Ansprüche, wobei das Element mit der Form eines Umfangsbogens (2) einen Querschnitt aufweist, der ausgewählt ist aus einer ellipsenförmigen, kreisförmigen, dreieckigen, viereckigen, sechseckigen, halbtrapezförmigen oder ähnlichen Form.

13. Implantat nach einem der vorstehenden Ansprüche, wobei der inerte oder biokompatible Polymerwerkstoff Polymethylmethacrylat umfasst.

14. Implantat nach einem der vorstehenden Ansprüche, wobei die vorgegebene Winkelerstreckung 90° beträgt.

## Revendications

1. Insert cornéen intrastromal (1), comprenant un élément ayant la forme d'un arc de circonférence (2) avec une extension angulaire prédéterminée (A) inférieure à 360° et réalisé en matériau polymère inerte ou biocompatible, destiné à être implanté dans le stroma de la cornée pour corriger des défauts de vision, ledit élément ayant la forme d'un arc de circonférence (2) comprenant des premiers trous passants (3'), adaptés pour laisser passer des fluides organiques, ayant les axes respectifs perpendiculaires ou sensiblement perpendiculaires au plan défini par ledit élément ayant la forme d'un arc de circonférence (2), et des deuxièmes trous passants (3"), adaptés pour laisser passer des fluides organiques, ayant les axes respectifs parallèles ou sensiblement parallèles au plan défini par ledit élément ayant la forme d'un arc de circonférence (2), **caractérisé en ce que** lesdits premiers trous passants (3') sont agencés de manière alternée avec des deuxièmes trous passants (3"), et/ou lesdits premiers trous passants (3') sont en communication avec lesdits deuxièmes trous passants (3").

2. Insert selon la revendication 1, dans lequel lesdits premiers trous passants (3') et lesdits deuxièmes trous passants (3") communiquant entre eux sont prévus au milieu dudit élément ayant la forme d'un arc de circonférence (2).

3. Insert selon la revendication 1, dans lequel lesdits premiers trous passants (3') et lesdits deuxièmes trous passants (3") communiquant entre eux sont prévus sur toute l'extension dudit élément ayant la forme d'un arc de circonférence (2).

4. Insert selon une des revendications précédentes, dans lequel ledit élément ayant la forme d'un arc de circonférence (2) a une variable épaisseur (D1, D2, Dx, Dy, Dz) de sa première extrémité (4) à sa deuxième extrémité (5).

5. Insert selon la revendication 4, dans lequel ladite épaisseur (D1, D2, Dx, Dy, Dz) varie progressivement d'une manière linéaire, ou sensiblement linéaire, de ladite première extrémité (4) à ladite deuxième extrémité (5).

6. Insert selon la revendication précédente, dans lequel ledit élément ayant la forme d'un arc de circonférence (2) a une première épaisseur (D1) au niveau de ladite première extrémité (4), et une deuxième épaisseur (D2), inférieure à (D1), au niveau de ladite deuxième extrémité (5).

7. Insert selon la revendication 4, dans lequel ledit élément ayant la forme d'un arc de circonférence (2) a une première épaisseur (D1) sensiblement en son milieu, et une deuxième épaisseur (D2), inférieure à (D1), au niveau de ladite première extrémité (4) et de ladite deuxième extrémité (5).

8. Insert selon l'une quelconque des revendications précédentes, dans lequel lesdits premiers trous passants (3') et lesdits deuxièmes trous passants (3") sont uniformément espacés les uns des autres.

9. Insert selon une des revendications 4-7, dans lequel lesdits trous passants (3',3") sont distribués de manière plus dense dans la zone dudit élément ayant la forme d'un arc de circonférence (2) ayant une épaisseur supérieure (D1, D2, Dx, Dy, Dz), ou dans la zone dudit élément ayant la forme d'un arc de circonférence (2) ayant une épaisseur inférieure (D1, D2, Dx, Dy, Dz).

10. Insert selon l'une quelconque des revendications précédentes, dans lequel lesdits trous passants (3';3") ont un diamètre entre 0,1 mm et 0,3 mm.

11. Insert selon l'une quelconque des revendications précédentes, dans lequel ledit élément ayant la forme d'un arc de circonférence (2) comprend au moins une ouverture d'extrémité (6), prévue au niveau de ladite première extrémité (4) et/ou ladite deuxième extrémité (5), adaptée pour un outil pour manipuler l'insert à insérer.

12. Insert selon l'une quelconque des revendications précédentes, dans lequel ledit élément ayant la forme d'un arc de circonférence (2) a une section transversale avec une forme sélectionnée parmi elliptique, circulaire, triangulaire, quadrangulaire, hexagonale, semi-trapézoïdale et similaire.

13. Insert selon l'une quelconque des revendications précédentes, dans lequel ledit matériau polymère inerte ou biocompatible comprend le polyméthacrylate de méthyle.

14. Insert selon l'une quelconque des revendications précédentes, dans lequel ladite extension angulaire prédéterminée est de 90°.
